## Europäisches Patentamt
## European Patent Office
## Office européen des brevets

(19)

(11) Veröffentlichungsnummer: **0 006 140**
**B1**

(12) **EUROPÄISCHE PATENTSCHRIFT**

(45) Veröffentlichungstag der Patentschrift: **01.04.81**

(21) Anmeldenummer: **79101560.5**

(22) Anmeldetag: **22.05.79**

(51) Int. Cl.³: **C 07 C 102/08,
C 07 C 103/167**

(54) Verfahren zur Herstellung von alpha-Hydroxycarbonsäureamiden.

(30) Priorität: **08.06.78 DE 2825267**

(43) Veröffentlichungstag der Anmeldung:
**09.01.80 Patentblatt 80/1**

(45) Bekanntmachung des Hinweises auf die Patenterteilung:
**01.04.81 Patentblatt 81/13**

(84) Benannte Vertragsstaaten:
**AT DE FR GB IT NL**

(56) Entgegenhaltungen:
**DE - A - 2 527 120
US - A - 3 166 588**

(73) Patentinhaber: **CHEMIE LINZ
AKTIENGESELLSCHAFT
St. Peter-Strasse 25
A-4020 Linz (AT)**
(73) Patentinhaber: **Lentia Gesellschaft mit
beschränkter Haftung
Schwanthalerstrasse 39 Postfach 20 16 26
D-8000 München 2 (DE)**

(72) Erfinder: **Wechsberg, Manfred, Dr.
Im Blumengrund 9/35
4020 Linz (AT)**
Erfinder: **Schönbeck, Rupert, Dr.
Ortmayrstrasse 26
4060 Leonding (AT)**

Courier Press, Leamington Spa, England.

EP 0 006 140 B1

Verfahren zur Herstellung von α-Hydroxycarbonsäureamiden

Die vorliegende Erfindung betrifft ein Verfahren zur Herstellung von α-Hydroxycarbonsäureamiden durch katalytische Anlagerung von Wasser an Cyanhydrine in Gegenwart von sauerstoffhaltigen Borverbindungen.

Die Addition von Wasser an Carbonsäurenitrile zu Carbonsäureamiden mit Hilfe von konzentrierter Schwefelsäure ist seit langem bekannt und wird beispielsweise bei der Herstellung von Acrylamid aus Acrylnitril großtechnisch verwendet. Da jedoch hiefür mehr als 1 Mol Schwefelsäure pro Mol Nitril verwendet werden muß, und außerdem intermediär eine salzartige Additionsverbindung entsteht, aus der das Amid durch Neutralisation der Mineralsäure isoliert werden muß, wurden in letzter Zeit katalytische Verfahren zur direkten Herstellung von Carbonsäureamiden aus Nitrilen entwickelt. Hierbei haben sich besonders Katalysatoren auf Basis von Kupfer, wie Kupfer-Chromoxyde, Kupfer-Molybdänoxyde, Kupferoxyde oder Raney-Kupfer bewährt und besitzen bereits großtechnische Bedeutung (Kirk-Othmer, Vol. 1 (1978), 303).

Will man jedoch aus einem α-Hydroxynitril das entsprechende α-Hydroxyamid darstellen, so versagen auch die wirksamsten dieser Kupferkatalysatoren. Lediglich Braunstein liefert gemäß den beiden Patentschriften DE—PS 15 93 320 und DE—PS 21 31 813 für den Fall der Hydratisierung von Acetoncyanhydrin einigermaßen brauchbare Ergebnisse. Aus der DE—OS 25 27 120, welche die Hydratation von Acetoncyanhydrin in Gegenwart von Braunstein betrifft, geht allerdings hervor, daß die katalytische Aktivität von Braunstein mehr oder weniger abhängig von seiner Modifikation ist. Namentlich δ-Braunstein, eine weitgehend amorphe Braunstein-Modifikation, ist danach besonders gut geeignet, doch erscheint der Einsatz dieses kommerziell nicht erhältlichen δ-Braunsteines, dessen Herstellung nach einer Vorschrift von O. Glemser et al., Z. anorg. allg. Chemie, *309* (1961/12), aus Kaliumpermanganat und Wasserstoffperoxyd erfolgt, erheblichen Aufwand mit sich zu bringen. Abgesehen von der relativ aufwendigen Herstellung des Katalysators ist eine heterogene Katalyse immer auch mit Problemen der Abtrennung des feinverteilten Katalysators aus der Reaktionslösung (z.B. durch Filtration) oder z.B. auch mit Erosionsproblemen während der Reaktion behaftet. Diese Hydratation wird entweder in Aceton oder in Gegenwart von Aceton durchgeführt.

Überraschenderweise konnte nun gefunden werden, daß die Wasseranlagerung an Cyanhydrine unter Bildung von α-Hydroxycarbonsäureamiden in Gegenwart einfacher, oft großtechnisch hergestellter Borverbindungen, wie Borax, Perborax oder anderen Salzen der Orthoborsäure, wie z.B. Alkali-, Erdalkalisalze oder Salze mit organischen Aminen in homogener Katalyse möglich ist.

Gegenstand der vorliegenden Erfindung ist demnach ein Verfahren zur Herstellung von α-Hydroxycarbonsäureamiden aus Cyanhydrinen durch katalytische Wasseranlagerung, das dadurch gekennzeichnet ist, daß die Wasseranlagerung in einem wäßrigen Reaktionsmedium, welches zwischen 10 und 60 Gewichtsprozent der dem Cyanhydrin zugrundeliegenden Oxoverbindung enthält, in Gegenwart eines im Reaktionsgemisch gelösten Katalysators, bestehend aus sauerstoffhaltigen Borverbindungen, bei einem pH-Wert von 7 bis 11 durchgeführt wird.

Die als Katalysator verwendeten, sauerstoffhaltigen Borverbindungen werden bei einem pH-Wert von 7 bis 11 eingesetzt, da sie im sauren Bereich ihre Wirksamkeit verlieren. Man verwendet Alkali- oder Erdalkalisalze der Ortho- oder Metaborsäure, wie zum Beispiel Natriumborate, Kaliumborate, Calciumborate. Bevorzugt sind großtechnisch hergestellte Salze, wie Borax oder Perborax. Man kann solche Salze jedoch auch in situ erzeugen, indem man Borsäure selbst durch Zugabe von Natron- oder Kalilauge (die Wahl der Base wird u.a. auch von den Löslichkeitseigenschaften des gebildeten Borates abhängen) auf den für die Hydratisierung des betreffenden Cyanhydrins optimalen pH-Wert einstellt. Bevorzugt sind Alkalisalze, die aus einer Mischung von Borsäure mit 5 bis 80 Gew.% NaOH bestehen. Besonders gute Ergebnisse werden auch bei Verwendung von Aminsalzen der Orthoborsäure erzielt. Hier wird ebenfalls die Borsäure mit dem entsprechenden Amin versetzt, bis der gewünschte pH-Wert erreicht ist. Als Amine eignen sich besonders tertiäre oder sekundäre Alkylamine, zweckmäßigerweise solche mit Alkylresten von 1—6 C-Atomen, wie z.B. Triäthylamin, Diäthylamin, Dimethylamin oder Di- bzw. Triisopropylamin. Bei Verwendung von Borax oder anderen Boraten, die in wäßriger Lösung alkalische Reaktion zeigen, kann eine Nacheinstellung des pH im allgemeinen unterbleiben. Etwaige Nebenreaktionen werden weitgehend dadurch unterdrückt, daß man dem Reaktionsgemisch eine Oxoverbindung, die dem Cyanhydrin zugrundeliegt, zugibt. Das Gewichtsverhältnis Oxoverbindung zu Wasser kann beliebig gewählt werden, solange eine ausreichende Löslichkeit des Katalysators gegeben und die Konzentration nicht so klein ist, daß technisch unbrauchbare Raum-Zeit-Ausbeuten resultieren. Man arbeitet in einem Reaktionsmedium, das 10 bis 60 Gew.% Oxoverbindung enthält.

Die übrigen Reaktionsbedingungen, wie Temperatur und Konzentrationsverhältnisse der Reaktionsteilnehmer können in einem entsprechenden Bereich so gewählt werden, daß sich der Katalysator im Reaktionsgemisch löst und ein Optimum an Umsatz und Ausbeute sowie Reinheit des gewünschten Amids erreicht wird.

Zweckmäßigerweise wird die Wasseranlagerung in flüssiger Phase bei Temperaturen zwischen 30 und 100°C erfolgen, wobei Temperaturen zwischen 50 und 70°C besonders bevorzugt sind. Besonders bevorzugt ist est weiter, den Katalysator in einer Konzentration von 0,1 bis 5 Gew.% zu verwenden.

Zur Durchführung des erfindungsgemäßen Verfahrens gibt man zu einer Mischung aus Cyanhydrin, Wasser und Oxoverbindung die geeignete Menge Borsäureverbindung und erwärmt das Reaktionsgemisch auf Reaktionstemperatur. Verwendet man z.B. Borsäure als Katalysator, so wird diese in Wasser gelöst und mit einer Base ein pH-Wert zwischen 7 und 11 eingestellt. Nach erfolgter Umsetzung destilliert man aus dem Reaktionsmedium die flüchtigen Bestandteile, nämlich Oxoverbindung, Wasser und nicht umgesetztes Cyanhydrin, ab, und verwendet sie für den nächsten Ansatz, während der Rückstand mit einem organischen Lösungsmittel, wie beispielsweise Aceton, extrahiert wird. Der hierbei entstehende Extraktionsrückstand stellt im wesentlichen die als Katalysator eingesetzte Borverbindung dar und kann für weitere Umsetzungen wiederverwendet werden, während das extrahierte α-Hydroxycarbonsäureamid nach Verdampfen des Extraktionsmittels direkt weiterverwendet oder durch Umkristallisation gereinigt werden kann.

Die Bedeutung des vorliegenden Verfahrens ist darin zu sehen, daß mit Hilfe eines sehr einfachen und billigen Katalysators in homogener Katalyse, also ohne Filtrations-schritte von Cu-Verbindungen oder δ-Braunstein aus einem Cyanhydrin, wie z.B. Acetoncyanhydrin, das α-Hydroxyisobutyramid hergestellt werden kann, aus dem nach bekannten Verfahren unter Veresterung und Dehydratisierung Methacrylsäureester hergestellt werden können (vgl. DE—OS 24 54 497) und DE—PS 12 11 153). Auch die übrigen, gemäß dem erfindungsgemäßen Verfahren herstellbaren Amide sind wertvolle Zwischenprodukte. Sie können z.B. zur Gewinnung verschiedener α-Hydroxy- sowie α-β-ungesättigter Carbonsäurederivate verwendet werden.

Im folgenden werden mehrere Beispiele beschrieben, die das erfindungsgemäße Verfahren näher erläutern. Die angeführten Beispiele sind jedoch keineswegs dazu geeignet, das Ausmaß der Erfindung zu begrenzen.

### Beispiel 1

Zu einer Lösung von 125 g (1,47 Mol) Acetoncyanhydrin in 300 g Wasser und 300 g Aceton wurden 11 g bei 80°C getrocknetes Borax (Gehalt an Bor 15,0%) zugegeben, das sich nach dem Erwärmen der Reaktionsmischung auf 65°C darin vollständig löste. Nach einer Reaktionszeit von 25 Stunden bei 65°C wurden die flüchtigen Anteile (bei 70°C und 3 000 Pa) abdestilliert, das erhaltene Destillat, das die restlichen, nicht umgesetzten Anteile an Acetoncyanhydrin enthielt, für den nächsten Ansatz bereitgestellt und der Rückstand bei 56°C mit insgesamt 500 ml Aceton extrahiert, wobei ein in Aceton unlöslicher Rückstand zurückblieb (10,5 g; Borgehalt 15,8%), der erneut als Katalysator für den nächsten Ansatz verwendet wurde. Die Acetonlösung wurde anschließend eingedampft, wobei 127,5 g an rohem α-Hydroxyisobutyramid vom Fp = 83—91°C zurückblieb. Das entspricht einer Rohausbeute, bezogen auf umgesetztes Acetoncyanhydrin, vom 92,4% den theorie.

Nach Reinigung durch Umkristallisation aus Aceton erhält man reines α-Hydroxyisobutyramid vom Fp = 90—94°C. Die Ausbeute beträgt 107 g, das sind 77,5% der Theorie.

### Beispiel 2

Ganz analog zu Beispiel 1 wurde ein weiterer Ansatz mit dem in Aceton unlöslichen Rückstand aus Beispiel 1 (10,5 g; 15,8% Bor) als Katalysator gefahren. Die Rohausbeute betrug bei diesem Folge-Ansatz, der unter gleichen Versuchsbedingungen wie in Beispiel 1 durchgeführt wurde, 135 g, das sind 96,4% der Theorie.

Nach Reinigung durch Umkristallisation wurden 110 g, das sind 78,5% der Theorie, reines α-Hydroxyisobutyramid, Fp = 90—94°C, erhalten. Nicht umgesetztes Acetoncyanhydrin wurde im abdestillierten Aceton/Wasser-Gemisch zurückgewonnen.

### Beispiel 3

125 g Acetoncyanhydrin und 23,5 g Natriumperborat wurden in 300 g Aceton und 300 g Wasser gelöst und 25 Stunden lang auf 60°C erwärmt. Der Cyanid gehalte der Reaktionslösung bei Titration nach Liebig betrug danach 0,3 mmol/ml gegenüber 1,84 mmol/ml zu Beginn der Umsetzung, was einem Umsetzungsgrad von 90% entspricht. Nach dem Abdestillieren der flüchtigen Anteile bei 90°C und 200 Pa wurde der Rückstand bei 50°C 3mal mit insgesamt 500 ml Aceton behandelt und die filtrierte Extraktionslösung bei 90°C und 200 Pa zur Trockene eingeengt. Als Rückstand wurden 120 g rohes α-Hydroxyisobutyramid erhalten, das entspricht 88% Ausbeute der Theorie, die durch Destillation oder Umkristallisation gereinigt werden können.

### Beispiel 4

Zu der Lösung aus 99 g Methyläthylketoncyanhydrin und 200 g Methyläthylketon wurden 200 g Wasser und 10 g Borax gegeben und das Gemisch 25 Stunden auf 60°C erwärmt. Danach wurden die Leichtsieder bei 90°C und 30 mbar abdestilliert und der Rückstand mit Aceton extrahiert. Die weitere Aufarbeitung erfolgte durch Destillation im Vakuum, wobei bei 97—104°C und 1 mbar 67 g 2-Hydroxy-2-methylbuttersäureamid erhalten wurden; das entspricht einer Ausbeute von 82% der Theorie, bezogen auf umgesetztes Cyanhydrin.

### Beispiel 5

Zu einer Lösung von 125 g Acetoncyanhydrin, 300 g Wasser und 300 g Aceton wurden 11 g Borsäure zugegeben und mit 3,5 g Kaliumhydroxyd ein pH-Wert von 8,9 eingestellt. Nach 25 Stunden bei 65°C wurde die Reaktion bei einem Umsetzungsgrad von 90,3% abgebrochen und die flüchtigen Anteile einschießlich des nicht umgesetzten Acetoncyanhydrins bei 3000 Pa abdestilliert. Der Rückstand wurde in 400 g Aceton aufgenommen, filtriert und das Aceton abdestilliert. Das so erhaltene Rohprodukt (136 g) wurde zur Reinigung im Vakuum destilliert. Die Ausbeute betrug 116 g, das entspricht 84,7% der Theorie von 136,8 g. Laut $NH_3$-Analyse war das $\alpha$-Hydroxyisobutyramid 97,1%ig.

### Beispiel 6

Zu 125 g Acetoncyanhydrin, 300 g Wasser und 300 g Aceton wurden 11 g Borsäure zugegeben und das Gemisch mit 3,2 g Ätznatron auf einen pH-Wert von 9 gestellt. Nach einer Reaktionszeit von 25 Stunden bei 65°C wurden die flüchtigen Anteile bei 3000 Pa abdestilliert und der Rückstand mit 400 g Aceton extrahiert. Die Rohausbeute an $\alpha$-Hydroxyisobutyramid betrug 130 g, das entspricht 96% der Theorie von 135,4 g, bezogen auf 89,4%igen Umsatz. Das Rohprodukt war laut $NH_3$-Bestimmung 91,8%.

### Beispiel 7

In 125 g (1,47 mol) Acetoncyanhydrin, 300 g Aceton und 300 g Wasser wurden 75 g Borsäure (Gehalt an Bor 17,47%) gegeben und 57 g Triäthylamin hinzugefügt. Die erhaltene Lösungwurde dann erwärmt und 14 Stunden bei 65°C gehalten, wonach 95,35% des eingesetzten Acetoncyanhydrins umgesetzt waren. Anschließend wurden die flüchtigen Anteile der Reaktionslösung im Vakuum abdestilliert und der verbleibende feste Rückstand mit 500 ml Aceton behandelt. Die acetonische Lösung wurde eingedampft und 153 g sandfarbener Rückstand erhalten, der laut Ammoniakbestimmung 94,1% $\alpha$-Hydroxyisobutyramid enthielt. Die Reinigung dieses Rohproduktes erfolgte durch zweimaliges Umkristallisieren aus Aceton. Hierbei wurden 103 g weiße Kristalle mit einem Schmelzpunkt von 90—93°C erhalten, das entspricht einer Selektivität von 72%. Laut Ammoniak-Wert war das Produkt 99,6%iges $\alpha$-Hydroxyisobutyramid.

### Patentansprüche

1. Verfahren zur Herstellung von $\alpha$-Hydroxycarbonsäureamiden aus Cyanhydrinen durch katalytische Wasseranlagerung, dadurch gekennzeichnet, daß die Wasseranlagerung in einem wäßrigen Reaktionsmedium, welches zwischen 10 und 60 Gew.% der dem Cyanhydrin zugrunde liegenden Oxoverbindung enthält, in Gegenwart eines im Reaktionsgemisch gelösten Katalysators, bestehend aus sauerstoffhaltigen Borverbindungen, bei einem pH-Wert von 7 bis 11 durchgeführt wird.

2. Verfahren nach Anspruch 1, dadurch gekennzeichnet, daß die sauerstoffhaltigen Borverbindungen Alkali- oder Erdalkalisalze der Orthoborsäure oder deren Salze mit organischen Aminen sind.

3. Verfahren nach den Ansprüchen 1 und 2, dadurch gekennzeichnet, daß die Alkalisalze aus einer Mischung von Borsäure mit 5 bis 80 Gew.% NaOH bestehen.

4. Verfahren nach den Ansprüchen 1 und 2, dadurch gekennzeichnet, daß die Alkalisalze Borax oder Perborax sind.

5. Vorfahren nach den Ansprüchen 1 und 2, dadurch gekennzeichnet, daß die Salze der Orthoborsäure mit organischen Aminen das Diaethylamin- oder Triaethylaminsalz der Orthoborsäure sind.

6. Verfahren nach den Ansprüchen 1 bis 5, dadurch gekennzeichnet, daß der Katalysator in einer Konzentration von 0,1 bis 5 Gew.% verwendet wird.

7. Verfahren nach den Ansprüchen 1 bis 6, dadurch gekennzeichnet, daß die Wasseranlagerung in flüssiger Phase bei Temperaturen zwischen 30 und 100°C erfolgt.

8. Verfahren nach den Ansprüchen 1 bis 6, dadurch gekennzeichnet, daß die Wasseranlagerung in flüssiger Phase bei Temperaturen zwischen 50 und 70°C erfolgt.

9. Verfahren nach den Ansprüchen 1 bis 7, dadurch gekennzeichnet, daß als Cyanhydrine solche Cyanhydrine verwendet werden, die sich von aliphatischen Ketonen oder Aldehyden ableiten.

10. Verfahren nach den Ansprüchen 1 bis 7, dadurch gekennzeichnet, daß als Cyanhydrine solche Cyanhydrine verwendet werden, die sich von aromatischen Aldehyden ableiten.

### Claims

1. A process for the preparation of $\alpha$-hydroxycarboxylic acid amides from cyanohydrins by catalytic addition reaction with water, characterised in that the addition reaction with water is carried out in an aqueous reaction medium, which contains between 10 and 60% by weight of the oxo compound on which the cyanohydrin is based, in the presence of a catalyst which is dissolved in the reaction mixture and consists of oxygen-containing boron compounds, at a pH value of 7 to 11.

2. A process as claimed in claim 1, wherein the oxygen-containing boron compounds are alkali metal salts or alkaline earth metal salts of orthoboric acid, or salts of orthoboric acid with organic amines.

3. Process according to Claims 1 and 2, characterised in that the alkali metal salts consist of a mixture of boric acid with 5 to 80% by weight of NaOH.

4. Process according to Claims 1 and 2, characterised in that the alkali metal salts are borax or perborax.

5. Process according to Claims 1 and 2, characterised in that the salts of orthoboric acid with organic amines are the diethylamine salt or triethylamine salt of orthoboric acid.

6. Process according to Claims 1 to 5, characterised in that the the catalyst is used in a concentration of 0.1 to 5% by weight.

7. Process according to Claims 1 to 6, characterised in that the addition reaction with water takes place in the liquid phase at temperatures of between 30 and 100°C.

8. Process according to Claims 1 to 6, characterised in that the addition reaction with water takes place in the liquid phase at temperatures of between 50 and 70°C.

9. Process according to Claims 1 to 7, characterised in that the cyanohydrins used are those which are derived from aliphatic ketones or aldehydes.

10. Process according to Claims 1 to 7, characterised in that the cyanohydrins used are those which are derived from aromatic aldehydes.

## Revendications

1. Procédé de préparation d'amides d'acides α-hydroxycarboxylliques à partir de cyanhydrines par fixation d'eau par voie catalytique, caractérisé en ce qu'on effectue la fixation d'eau dans un milieu de réaction aqueux renfermant entre 10 et 60% en poids du composé oxo qui est à la base de la cyanhydrine, en présence d'un catalyseur dissous dans le mélange réactionnel, constitué par des composés oxygénés du bore, à une valeur de pH allant de 7 à 11.

2. Procédé suivant la revendication 1, caractérisé en cel que les composés oxygénés du bore sont des sels alcalins ou alcalino-terreux de l'acid orthoborique ou ses sels avec des amines organiques.

3. Procédé suivant les revendications 1 et 2, caractérisé en ce que les sels alcalins sont constitués par un mélange d'acide borique avec 5 à 80% en poids de NaOH.

4. Procédé suivant les revendications 1 et 2, caractérisé en ce que les sels alcalins sont du borax ou de perborax.

5. Procédé suivant les revendications 1 et 2, caractérisé en ce que les sels de l'acide orthoborique avec des amines organiques sont le sel de diéthylamine ou de triéthylamine de l'acide orthoborique.

6. Procédé suivant l'une quelconque des revendications 1 à 5, caractérisé en ce que le catalyseur est utilisé selon une concentration de 0,1 à 5% en poids.

7. Procédé suivant l'une quelconque des revendications 1 à 6,, caractérisé en ce que la fixation d'eau est effectuée en phase liquide à des températures comprises entre 30 et 100°C.

8. Procédé suivant l'une quelconque des revendications 1 à 6, caractérisé en ce que la fixation d'eau est effectuée en phase liquide à des températures comprises entre 50 et 70°C.

9. Procédé suivant l'une quelconque des revendications 1 à 7, caractérisé en ce qu'on utilise comme cyanhydrines des cyanhydrines qui sont dérivés de cétones ou d'aldéhydes aliphatiques.

10. Procédé suivant l'une quelconque des revendications 1 à 7, caractérisé en ce qu'on utilise comme cyanhydrines des cyanhydrines dérivés d'aldéhydes aromatiques.